# EUROPEAN PATENT SPECIFICATION

(11) **EP 1 874 199 B1**
(45) Date of publication and mention of the grant of the patent: **24.06.2009**
(21) Application number: 06728540.3
(22) Date of filing: 26.04.2006
(51) Int. Cl.: A61B 17/34, A61B 17/02, A61B 1/32

(54) **RADIALLY EXPANDABLE ANCHORAGE GUIDE FOR TROCARS**
RADIAL EXPANDIERBARE VERANKERUNGSFÜHRUNG FÜR TROKARE
GUIDE D'ANCRAGE RADIALEMENT EXTENSIBLE POUR TROCARTS

(30) Priority: 29.04.2005 IT FI20050082
(43) Date of publication of application: 09.01.2008
(73) Proprietor: Elenor S.R.L., 52100 Arezzo (IT)
(72) Inventor: FERRARI, Danilo, I-52100 AREZZO (IT)
(74) Representative: Bardini, Marco Luigi
(86) International application number: PCT/IT2006/000283
(87) International publication number: WO 2006/117819

(56) References cited:
- WO-A-99/52577
- GB-A- 330 629
- US-A- 3 789 852
- US-A- 5 871 474
- US-A1- 2004 215 063

## Description

### Field of the Invention

The present invention regards in general the field of equipment for laparoscopic surgery and more in particular refers to a radially expandable anchorage guide for trocars.

### Background Art

As is known, in the laparoscopic surgery field wide use is made of instruments called trocars which generate and maintain the access channels for the various surgical instruments used in operations. Schematically, a commercial trocar comprises a cannula and a valve body situated at one of its ends. Typically, the cannula has a diameter of 5 or 12 mm with a length of 110-120 mm.

At the beginning of the operation, some holes are made, for example, in the abdomen of the patient by using as many trocars equipped with an accessory capable of penetrating the various tissue layers. Subsequently, such accessory is extracted and an inert gas is insufflated in the abdomen through one of the trocars so as to generate the necessary operating space.

The pressure generated inside the abdomen tends to push the trocars outwardly, so that various methods have been devised for their anchorage. The most widespread solution foresees the use of a tube with inner diameter equal to the outer diameter of the trocar cannula and with an outer threaded surface such to permit the surgeon to "screw it" into the access hole. The tube is then fixed to the cannula of the trocar by means of elastic bands or friction systems.

During the operation, all of the necessary instruments are inserted through the trocars. In the case in which internal parts must be removed, for example gall bladder, intestine parts, tumoral masses etc., it may become necessary to carry out further access cuts of sufficient size for the passage of the part to be removed. This need involves making additional wounds of greater size than those left by the trocars, as well as the possibility of contamination through the walls of the cut during the extraction step of the parts to be removed; all of this results in a more difficult patient recovery after the operation. After the extraction, it is often necessary to continue to operate laparoscopically and the additional cut compromises the gas seal so that it is necessary to use instruments capable of restoring the seal.

GB 330629 discloses an expansible speculum according to the preamble of claim 1 comprising a number of blades extending axially from a housing containing a mechanism adapted for simultaneously operating the blades to spread them in several directions from the axis, so that a passage, into which said blades are inserted, is uniformly expanded. The mechanism for spreading the blades is of the type in which the operation of a screw or knob in a circular direction effects an equal and parallel outward movement of all the blades.

US 3789852 discloses an expandable tubular element, especially for use as a trochar, in which one of more thin metal strips, such as stainless steel, are arranged to form a tubular element which can be expanded in the radial direction. In particular, the tubular element comprises a strip of stainless steel wound up spirally to form a tube with the free ends of the thus wound up tube being movable in the circumferential direction to cause expansion and contraction of the trochar.

### Summary of the invention

The object of the present invention is to provide an anchorage guide for a trocar which, taking advantage of the elasticity of the relaxed tissues (since they are anesthetized), can expand to generate the necessary space for the extraction of the parts to be removed without the need to carry out additional cuts.

Another object of the present invention is to provide an anchorage guide for trocars of the type mentioned above on which it is possible to mount an autonomous valve system through which instruments or an adaptor for commercial trocars can pass, if, after the extraction of the diseased parts, it would be necessary to newly pressurize the abdomen and restart the operation.

These objects are achieved with the anchorage guide for a trocar according to the present invention, the main features of which are defined in claim 1.

### Brief description of the drawings

Other characteristics, as well as the advantages, of the anchorage guide for a trocar according to the present invention will be clearer from the following description of an exemplifying and not limiting embodiment with reference to the attached drawings wherein:
- Figure 1 is a perspective view of the anchorage guide according to the invention in its closed position;
- Figure 2 is a perspective view of the anchorage guide in its open position;
- Figure 3 is a cross sectional view of the anchorage guide of figure 1;
- Figure 4 is an exploded view of the anchorage guide according to the invention;
- Figure 5 is a perspective view of the anchorage guide according to the invention in a completely open position and with a diaphragm valve inserted therein;
- Figure 6 is a longitudinal sectional view of the anchorage guide of figure 5;
- Figure 7 is a longitudinal sectional view of the anchorage guide according to the invention in its closed position with a commercial valve system;
- Figure 8 is a perspective view of the anchorage guide according to the invention in is its closed position, equipped with a diaphragm valve with a commercial trocar housed therein;
- Figure 9 shows a perspective view of the anchorage guide according to the invention with a protective tube.

### Detailed description of the invention

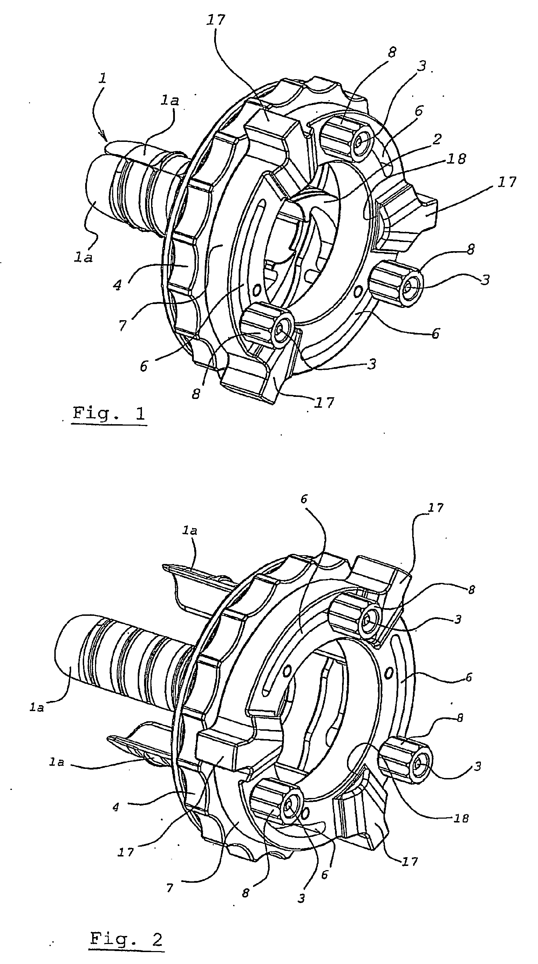

With reference to figures 1-4, a tubular body, generically indicated with 1, is formed by three sectors la of angular width equal to 120°. From one end of each of the sectors la, a curved arm 2 extends in a substantially tangential manner; the free end of the curved arm 2 is rotatably engaged with a pin 3. The three pins are in turn engaged in three equidistant holes 5 formed on a first ring nut 4, called fixed ring nut, orthogonal to the longitudinal axis of the tubular body 1 and in circumferential slots 6 made along a second ring nut 7, called moveable ring nut, coaxially arranged on the fixed ring nut 4. The threaded ends of the pins 3 projecting from the slots 6 of the moveable ring nut 7 are finally engaged in respective threaded knobs 8, abutting against moveable ring nut 7 opposedly with respect to the fixed ring nut 4, thereby obtaining the mutual fixing of the various components.

The three sectors la of the tubular body 1 form a channel of inner diameter less than or equal to the outer diameter of the cannula of the commercial trocar to be used, while the outer surface of the sectors la has a saw tooth thread, as in the anchorage cannulae of known type, capable of grasping the walls of the body cavity access hole.

The three curved arms 2 are housed within the fixed ring nut 4, coplanar thereto, and may be simultaneously rotated around the respective pins 3, to transmit an angular movement to the moveable ring nut 7. Following the rotation of the arms 2, the cylindrical sectors la extending therefrom progressively divaricate from each other, passing from the closed configuration illustrated in figure 1 to the open configuration of figure 2.

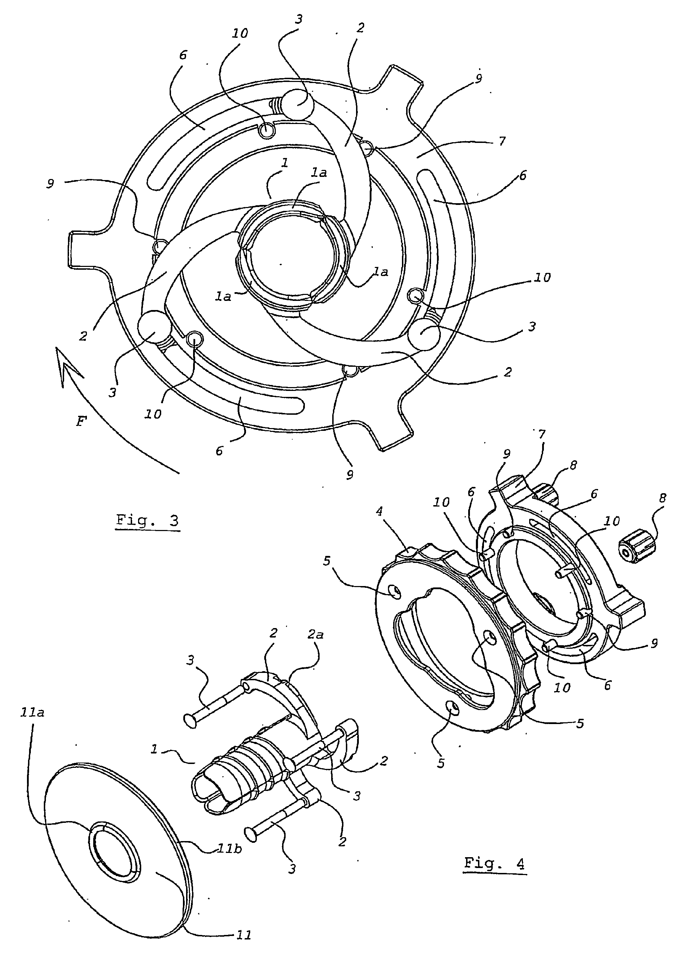

Three angularly equidistant closure pins 9 and three thrust pins 10 extend orthogonally from the face of the moveable ring nut 7 turned towards the fixed ring nut 4. When the tubular body 1 is in its closed position, as shown in figure 1, the closure pins 9 abut on the convex side of the respective curved arms 2 and maintain the three cylindrical sectors la adjacent to each other, tightening them on the cannula of the trocar so as to permit the axial locking of the trocar. In particular, as shown in figure 4, the arms 2 have seats 2a, within which the closure pins 9 are engaged. On the other hand, when the moveable ring nut 7 is rotated in the direction of the arrow F of figure 3, the thrust pins 10 come into contact with the concave profile of the arms 2 of the sectors la and, sliding along them away from the hinge pins 3, ensure that the arms progressively extend. The opening of the sectors 1a first occurs quickly and then slows approaching the end stop; in this way it is possible to exert a force as constant as possible throughout the opening step, since the resistance of the tissues increases as the divarication of the sectors la increases. At the stop end, the tubular body 1 remains stably open when the thrust pins reach the dead point. If it is necessary to stop the opening in an intermediate position, it is sufficient to tighten at least one of the three threaded knobs 8.

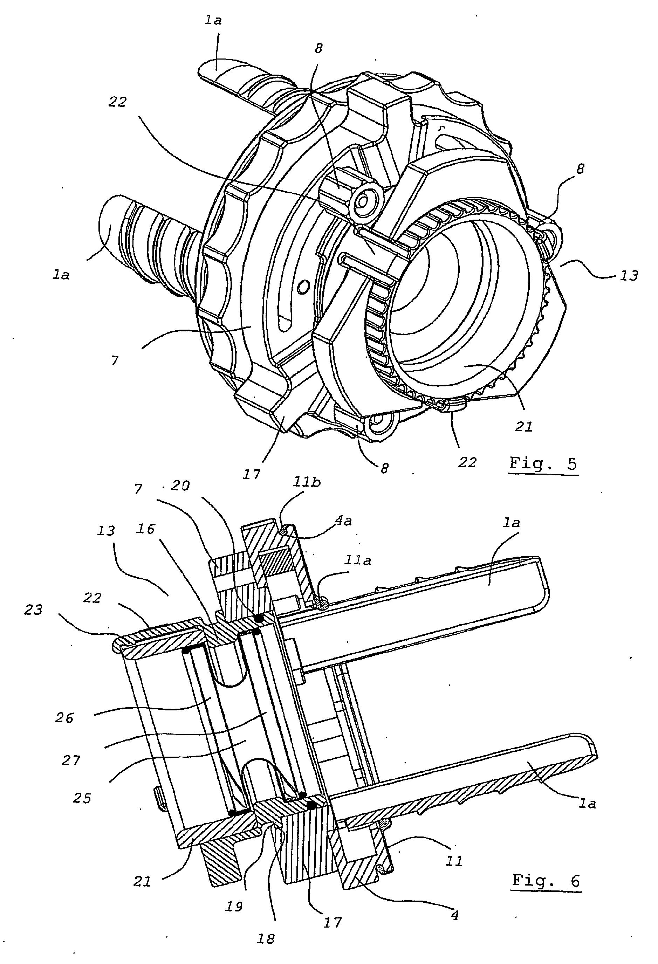

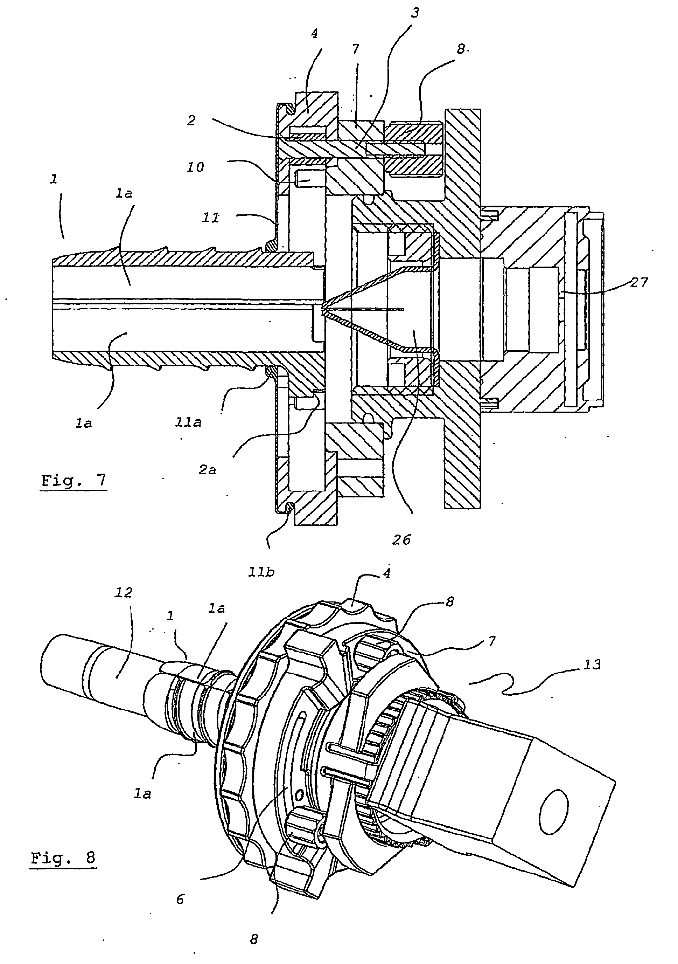

To ensure an adequate sealing of the gas on the patient side, a membrane 11 is foreseen as shown in figure 4 and figure 7, while on the surgeon's side the seal is ensured by a valve system such as that shown in figures 5 and 7. The membrane 11 has a variable thickness, and in particular in correspondence with its minimum and maximum diameter terminates with respective toroidal rings 11a and 11b. The maximum diameter ring 11b is inserted in a perimetrical groove 4a of the ring nut 4, as is visible in figure 6, while the minimum diameter ring 11a is tight at the base of the sectors 1a. No groove for housing the ring 11a is foreseen on said sectors, since the correct position is maintained both because it is a rest position and because the membrane is forced by the abdomen of the patient against the ring nut 4.

The radially expandable anchorage guide for trocars according to the present invention is used in the following manner. At the beginning of the operation, during the insertion step of the trocar in the abdomen (for example), the anchorage guide according to the present invention is used as if it was a normal anchorage tube of the trocar to the abdominal wall. As shown in figure 8, the three sectors la are closed around the cannula 12 of the trocar and are tightened to it by rotating the moveable ring nut 7 with respect to the fixed ring nut 4, and tightening at least one of the locking knobs 8. In the particular case wherein the trocar inserted is calibrated on the inner size of the closed tubular body 1, tightening the locking knob 8 can be avoided since the system is irreversible when in completely closed position. A valve 13, of the type illustrated in figure 5 and 6, is closed around the trocar tube to ensure a perfect gas seal. In the case in which the valve is that illustrated in said figures, it is not necessary to tighten the tubular body 1 on the cannula 12 of the trocar, since the same valve can ensure the axial anchorage of the trocar. Indeed, when the membrane tightens around the cannula of the trocar, it generates a consistent radial force which, due to the high coefficient of friction between the same membrane and the cannula of the trocar, ensures a strong axial seal.

If during the operation it becomes necessary to insert a trocar of greater size, it will suffice to open the valve 13 of figure 8, open the tubular body 1 by unlocking the cannula 12 of the trocar, extract the trocar to be substituted, insert the new trocar and tighten the tubular body 1 and the seal valve 13 on it.

If during the operation it becomes necessary to remove an internal mass, the tubular body 1 may be divaricated to its maximum expansion so that, once the trocar and the seal valve is removed, an access channel is generated at the abdomen of sufficient dimensions for the passage of the mass to be removed.

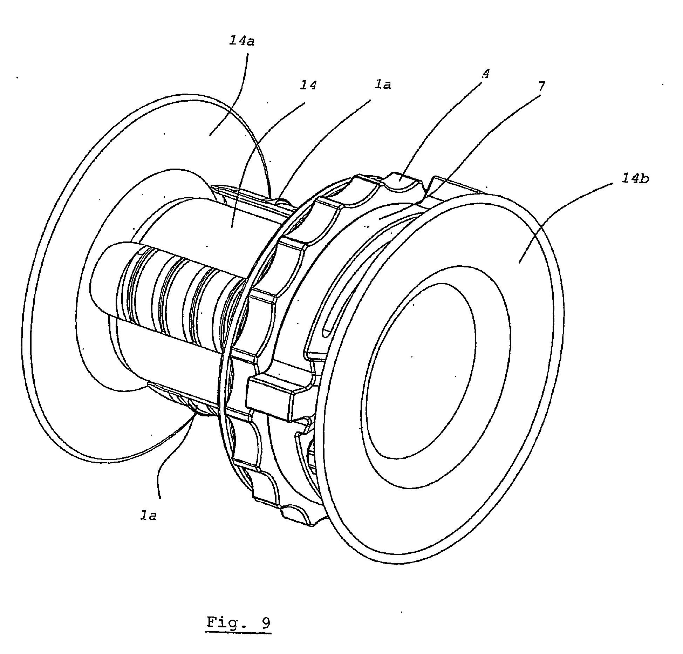

To protect the walls of the access hole from possible contaminations (for example during the extraction of a tumoral mass in the absence of other types of protection), it is possible to insert within the divaricated sectors la a tube 14 (see figure 9) after having overturned the elastic membrane 14a inside the tube itself. In particular, the tube 14 is composed of a rigid cylinder of thin thickness buried within an elastic membrane terminating at both ends with two large diameter discs with reinforced edges, one of which is the membrane 14a. For its insertion inside the divaricated tubular body 1, the inner disc or membrane 14a is folded inside the tube 14 and subsequently made to expand inside the abdomen. The membrane 14a protects from contaminations the inner wall of the abdomen near the access hole. The axial position of the tube 14 is ensured by the tightening of the three sectors la on it. The tube 14 has an outer elastic disc 15 at the other end which may be folded on the moveable ring nut 7 of the anchorage guide so as to protect it from contamination. Figure 9 has the expandable anchorage guide according to the invention in a configuration suitable for the extraction of an internal mass. To extract the tube 14, it suffices to further divaricate the sectors la and pull the tube 14 through the outer disc 15.

The valve 13 illustrated in figures 5 and 6 is based on the principal of operation of a commercial device named "LAP-DISC", described in the U.S. patents No. 6110154 and 6589167 and used for making an abdomen access for the surgeon's hand in hand-assisted laparoscopic surgery operations, even if it uses a different method for maintaining the set position.

With particular reference to figures 5 and 6, the valve 13 comprises a fixed support 16 which can be connected by bayonet coupling to the outer face of the moveable ring nut 7. On the latter, in fact, radial expansions 17 are foreseen defining circumferential grooves 18 with the outer face of the moveable ring nut 7, within which radial tongues 19 are friction engaged, extending from the outer wall of the fixed support 16. The seal between the fixed support 16 and the moveable ring nut 7 is ensured by a seal ring 20 arranged therebetween. The valve 13 moreover comprises a control ring nut 21 rotatably engaged within the fixed support 16 and maintained in the desired angular position by means of flexible arms 22 axially extending from the fixed support 16. The arms 22 have inner radial projections 23 which are engaged in axial grooves 24 formed on the outer surface of the control ring nut 21.

The obturator of the valve 13 comprises an elastic membrane 25 having in rest position a toroidal shape with "omega" cross section, which is maintained tight on the inner walls of the control ring nut 21 and the fixed support 16, respectively, by means of expansion rings 26 and 27 of rectangular section, cut sideways to permit the flattening of the membrane against the walls of the control ring nut 21 and the fixed support 16 without gap.

Rotating the control ring nut 21 with respect to the support 16, the flexible arms 22 bend, making the projections 23 move from one groove 24 to the other, so that the elastic membrane 25, due to the torsion to which it is subjected, closes radially like a diaphragm. With an appropriate rotation angle of the control ring nut 21, it is possible to completely occlude the opening of the valve 13, or partially occlude it in case the cannula of a trocar must pass through said opening, tightening the membrane 25 around it and ensuring the gas seal and a consistent axial tightening.

The expandable anchorage guide according to the invention, in addition to having the diaphragm valve described above, may naturally be employed even in association with valves for trocars of another type, such as that illustrated in figure 7.

The valve herein illustrated is of double seal type: i.e. it has a first elastic obturator 26 with flute mouth geometry which in rest is maintained in closed position by the pressure established in the body cavity. If an instrument is inserted, the flute mouth 26 obturator opens in correspondence with the longitudinal cuts, losing however the gas seal. A second obturator 27 is therefore foreseen, upstream of the first and formed by an elastic membrane with calibrated hole to make a seal on a particular instrument diameter. Normally, having to insert an instrument with different diameter, the second obturator block 27 fixed with bayonet coupling to the first obturator must be substituted. The double obturator scheme as illustrated allows instruments to be extracted and inserted without losing the seal, while, when the instrument is inserted, the second obturator ensures the seal by forcing radially against the instrument. The valve system described and illustrated in figure 7 is among the most commercially widespread, but there exist many others predominantly intended to house instruments with different diameters, without the need to interchange the second obturator. The instrument according to the invention may house, by means of an appropriate adaptor, any commercial sealing system.

Naturally, the tubular body 1 of the expandable anchorage guide according to the invention can be made in a different number of cylindrical sectors a with respect to that described and illustrated. In this manner, it is possible to better approximate the circular shape of the realized opening, through the cost of the instrument increases.

Variations and/or modifications can be made to the anchorage guide for trocars according to the present invention without departing from the protective scope of the invention as set forth in the following claims.

## Claims

1. Anchorage guide for trocar for use in laparoscopic surgery comprising a tubular body (1) formed by a plurality of substantially circular sectors (la), radially moveable from and towards a longitudinal axis of said body between a first position, wherein they flank each other according to a substantially circular first arrangement of diameter equal to that of said tubular body, and a second position, wherein they are equidistant from each other according to a substantially circular second arrangement of greater diameter than that of said first circular arrangement, anchorage means formed on the outer surface of said tubular body for securing said tubular body to an access hole for entering a body cavity, said plurality of sectors being rotatably connected with a support element (4) through respective arms (2) extending therefrom in a substantially tangential manner and manual operation means (7), moveably connected to said support element, being further provided for moving said sectors from said first to said second position and vice versa, said support element (4) being an annular body and **characterized in that** said manual operation means comprises a moveable ring nut (7) coaxially rotating on said annular body and thrust pins (10) extending orthogonally from said moveable ring nut (7) and arranged in a manner to interfere with said arms (2) during the rotation on said ring nut (7), the sliding of said thrust pins (10) along said arms (2) causing the angular displacement of said arms.

2. Anchorage guide according to claim 1, wherein said arms (2) have a curved profile.

3. Anchorage guide according to claim 1 or 2, wherein said manual operation means also comprise closure pins (9) extending from said moveable ring nut (7) and also adapted to interfere with said arms (2) from the opposite part of said thrust pins (10).

4. Anchorage guide according to any one of the previous claims, wherein said arms (2) are housed coplanarly within said annular body (4).

5. Anchorage guide according to any one of the previous claims, wherein said arms (2) are hinged to said support element (4) by means of respective hinge pins (3) passing through said support element and engaged in respective circumferential slots (6) formed along said moveable ring nut (7), reversible tightening means (8) of said pins abutting against said moveable ring nut.

6. Anchorage guide according to any one of the previous claims, wherein a tube (14) can be attached within said tubular body (1) in open condition, said tube having two elastic disc-shaped membranes (14a, 14b) extending from its ends and foldable within it.

7. Anchorage guide according to any one of the previous claims, wherein said moveable ring nut (7) comprises attachment means (17, 18) for a valve body (13).

8. Anchorage guide according to claim 7, wherein said valve body comprises a fixed support (16) which can be reversibly connected to said moveable ring nut (7) and a control ring nut (21) pivotally engaged in said fixed support (16) and maintained in a desired angular position by means of flexible arms (22) extending axially from the fixed support (16), said arms having inner radial projections (23) adapted to be engaged in axial grooves (24) formed on the edge of said control ring nut (21).

## Patentansprüche

1. Verankerungsführung für Trokare zur Verwendung bei laparoskopischer Chirurgie, enthaltend einen rohrartigen Körper (1), der durch eine Mehrzahl von im Wesentlichen kreisartigen Sektoren (1a) gebildet ist, die radial von und hin zu einer Längsachse des Körpers zwischen einer ersten Position, in der sie einander gemäß einer im Wesentliche kreisartigen ersten Anordnung eines Durchmessers gleich jenem des rohrartigen Körpers flankieren, und einer zweiten Position beweglich sind, in der sie gemäß einer im Wesentlichen kreisartigen zweiten Anordnung eines größeren Durchmessers als jener der ersten kreisartigen Anordnung voneinander gleich beabstandet sind, wobei Verankerungseinrichtungen an der äußeren Oberfläche des rohrartigen Körpers gebildet sind, um den rohrartigen Körper an einem Zugriffsloch zum Eindringen in einen Körperhohlraum zu befestigen, welche Mehrzahl von Sektoren drehbar mit einem Halteelement (4) durch entsprechende Arme (2) verbunden sind, die sich davon in einer im Wesentlichen tangentialen Weise erstrecken, und wobei ferner manuelle Betätigungseinrichtungen (7), die beweglich mit dem Halteelement verbunden sind, vorgesehen sind, um die Sektoren von der ersten zu der zweiten Position und umgekehrt zu bewegen, welches Halteelement (4) ein ringartiger Körper ist, und **dadurch gekennzeichnet, dass** die manuellen Betätigungseinrichtungen eine beweglich Ringbuchse (7), die sich koaxial auf dem ringartigen Körper dreht, und Schubstifte (10) enthalten, die sich orthogonal von der beweglichen Ringbuchse (7) erstrecken und in einer Weise angeordnet sind, um mit den Armen (2) während der Drehung der Ringbuchse (7) wechselzuwirken, wobei das Gleiten der Schubstifte (10) längs der Arme (2) die winkelmäßige Verstellung der Arme verursacht.

2. Verankerungsführung nach Anspruch 1, wobei die Arme (2) ein gekrümmtes Profil haben.

3. Verankerungsführung nach Anspruch 1 oder 2, wobei die manuellen Betätigungseinrichtungen auch Verschlussstifte (9) enthalten, die sich von der beweglichen Ringbuchse (7) aus erstrecken und auch ausgelegt sind, um mit den Armen (2) von dem entgegengesetzten Teil bezüglich der Schubstifte (10) wechselzuwirken.

4. Verankerungsführung nach einem der vorhergehenden Ansprüche, wobei die Arme (2) koplanar innerhalb des ringartigen Körpers (4) untergebracht sind.

5. Verankerungsführung nach einem der vorhergehenden Ansprüche, wobei die Arme (2) an dem Halteelement (4) mittels entsprechender Drehstifte (3) angelenkt sind, die durch das Halteelement hindurch gehen und in entsprechenden umfangsmäßigen Schlitzen (6) in Eingriff sind, die längs der beweglichen Ringbuchse (7) ausgebildet sind, wobei reversible Anzugseinrichtungen (8) der Stifte gegen die bewegliche Ringnut anliegen.

6. Verankerungsführung nach einem der vorhergehenden Ansprüche, wobei ein Rohr (14) innerhalb des rohrartigen Körpers (1) in einem offenen Zustand angebracht werden kann, welches Rohr zwei elastische scheibenförmige Membranen (14a, 14b) hat, die von seinen Enden ausgehen und in es hinein faltbar sind.

7. Verankerungsführung nach einem der vorhergehenden Ansprüche, wobei die bewegliche Ringbuchse (7) Befestigungseinrichtungen (17, 18) für einen Ventilkörper (13) hat.

8. Verankerungsführung nach Anspruch 7, wobei der Ventilkörper eine feste Halterung (16), die reversibel mit der beweglichen Ringbuchse (7) verbunden werden kann, und eine Steuerringbuchse (21) enthält, die drehbar in der festen Halterung (16) in Eingriff ist und in einer gewünschten Winkelposition mittels flexiblen Armen (22) gehalten wird, die sich axial von der festen Halterung (16) erstreckt, welche Arme innere radiale Vorsprünge (23) haben, die ausgelegt sind, um in axialen Nuten (24) in Eingriff zu sein, die an dem Rand der Steuerringbuchse (21) ausgebildet sind.

## Revendications

1. Guide d'ancrage pour trocart pour utilisation en chirurgie laparoscopique comprenant un corps tubulaire (1) constitué d'une pluralité de secteurs sensiblement circulaires (1a), pouvant être déplacés radialement à partir d'un et vers un axe longitudinal du dit corps entre une première position où ils viennent au contact les uns des autres par leur bords latéraux selon un premier agencement sensiblement circulaire de diamètre égal à celui du dit corps tubulaire, et une seconde position où ils sont équidistants entre eux selon un second agencement sensiblement circulaire de plus grand diamètre que celui du dit premier agencement circulaire, des moyens d'ancrage formés sur la surface extérieure du dit corps tubulaire pour fixer ledit corps tubulaire à un orifice d'accès pour pénétrer dans une cavité d'un corps, ladite pluralité des secteurs étant reliés de façon pivotante à un élément de support (4) via des bras respectifs (2) s'étendant à partir de celui-ci de façon sensiblement tangentielle, et des moyens de commande manuels (7), reliés de façon mobile au dit élément de support, étant en outre agencés pour déplacer lesdits secteurs de ladite première à ladite seconde position et vice versa, ledit l'élément de support (4) étant un corps annulaire, **caractérisé en ce que** lesdits moyens de commande manuels comprennent une bague en anneau mobile (7) tournant de façon coaxiale sur ledit corps annulaire et des doigts de poussée (10) se prolongeant orthogonalement à partir de la bague en anneau mobile (7) et agencés de façon à interférer avec lesdits bras (2) pendant la rotation de ladite bague en anneau (7), le glissement des doigts de poussée (10) le long des dits bras (2) entraînant le déplacement angulaire des dits bras.

2. Guide d'ancrage selon la revendication 1, où lesdits bras (2) ont un profil courbé.

3. Guide d'ancrage selon l'une des revendications 1 et 2, où lesdits moyens de commande manuels comportent aussi des doigts de fermeture (9) s'étendant à partir de ladite bague en anneau mobile (7) et également adaptés pour interférer avec lesdits bras (2) à l'opposé des dits doigts de poussée (10).

4. Guide d'ancrage selon l'une quelconque des revendications précédentes, où lesdits bras (2) sont logés de façon coplanaire dans ledit corps annulaire (4).

5. Guide d'ancrage selon l'une quelconque des revendications précédentes, où lesdits bras (2) sont articulés sur ledit élément de support (4) au moyen de doigts charnières respectifs (3) passant à travers ledit élément de support et engagés dans des fentes circulaires respectives (6) réalisées le long de ladite bague en anneau mobile (7), des moyens de serrage réversibles (8) des dits doigts butant contre ladite bague en anneau mobile.

6. Guide d'ancrage selon l'une quelconque des revendications précédentes, où un tube (14) peut être fixé dans ledit corps tubulaire (1) en état ouvert, ledit tube ayant deux membranes élastiques en forme de disque (14a, 14b) s'étendant à partir de ses extrémités et repliables à l'intérieur de celui-ci.

7. Guide d'ancrage selon l'une quelconque des revendications précédentes, où ladite bague en anneau mobile (7) comprend des moyens d'attache (17, 18) pour un corps de valve (13).

8. Guide d'ancrage selon la revendication 7, où ledit corps de valve comprend un support fixe (16) qui peut être relié de façon réversible à ladite bague en anneau mobile (7) et une bague de commande en anneau (21) montée de façon pivotante dans ledit support fixe (16) et maintenue dans une position angulaire désirée au moyen de bras flexibles (22) s'étendant axialement à partir du support fixe (16), lesdits bras ayant des projections radiales intérieures (23) adaptées pour être engagées dans des gorges axiales (24) réalisées sur le bord de ladite bague de commande en anneau (21).
